# EUROPEAN PATENT APPLICATION

(11) **EP 4 487 843 A1**
(43) Date of publication of application: **08.01.2025**
(21) Application number: 23763325.0
(22) Date of filing: 22.02.2023
(51) Int. Cl.: A61K 31/197, A61K 31/295, A61K 31/519, A61K 33/26, A61P 17/06, A61P 19/02, A61P 29/00, A61P 37/06, A61P 43/00

(54) **PHARMACEUTICAL COMPOSITION FOR PREVENTING AND/OR TREATING AUTOIMMUNE DISEASES**

(30) Priority: 01.03.2022 JP 2022030907
(71) Applicant: National Center for Child Health and Development, Tokyo 157-8535 (JP); SBI Pharmaceuticals Co., Ltd., Tokyo 106-6020 (JP)
(72) Inventor: RII, Ko, Tokyo 157-8535 (JP)
(74) Representative: Greaves Brewster LLP
(86) International application number: PCT/JP2023/006410
(87) International publication number: WO 2023/167066

(57) **Abstract**

The object of the invention is to search for novel drug or method for treating autoimmune diseases by means of a combination of methotrexate and 5-ALAs.

## Description

### Technical Field

The present invention relates to a pharmaceutical composition for preventing and/or treating autoimmune diseases.

### Background Art

An autoimmune disease is a disease that arises from the immune system attacking normal autologous cells or tissues due to an abnormality in the immune system, and various pathologies exist depending on the type of cell or tissue subject to attack. In the treatment of autoimmune diseases, it is important to suppress inflammation in particular, and immunosuppressing agents, anti-inflammatory agents, palliative care, and the like are generally employed.

Among autoimmune diseases, the number of rheumatoid arthritis (RA) patients is particularly high, having estimates of about 700 - 800 thousand in Japan and about 23 million in the world. It is known that most rheumatoid arthritis are related to autoimmunity, but their cause have not been elucidated in detail, and there are many things that are unclear (Non-Patent Literature 1) .

### Citation List

### Non-Patent Literatures

[Non-Patent Literature 1] Lancet. 2017 Jun 10; 389(10086): 2328 - 2337

### Summary of the Invention

### Problems to be Solved by the Invention

The object of the present invention is to search for a novel drug or a method for treating autoimmune diseases.

### Means for Solving the Problems

The present inventors found that by using methotrexate (MTX) and 5-ALAs in combination, the expression of anti-inflammatory proteins (such as HO-1) significantly increases at the cell level (i.e., in vitro) as well as at the living body level (i.e., in vivo). Based on the discovery of this novel action mechanism, the present inventors found that the therapeutic effect for autoimmune diseases may be synergistically elevated by using methotrexate (MTX) and 5-ALAs in combination, thus coming to complete the present invention.

In other words, in one embodiment, the present invention relates to a pharmaceutical composition for preventing and/or treating autoimmune diseases, characterized in that it comprises:
(A) a compound shown by the following Formula (I): 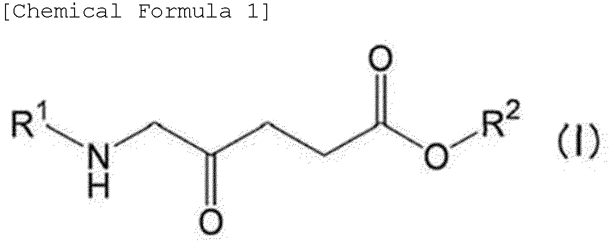 (wherein R¹ represents a hydrogen atom or an acyl group, and R² represents a hydrogen atom, a linear or branched alkyl group, a cycloalkyl group, an aryl group, or an aralkyl group) or a pharmaceutically acceptable salt or ester thereof, and
(B) methotrexate or a pharmaceutically acceptable salt thereof.

Another embodiment of the present invention relates to a pharmaceutical composition for preventing and/or treating autoimmune diseases, characterized in that the pharmaceutical composition comprises:
a compound shown by the following Formula (I): 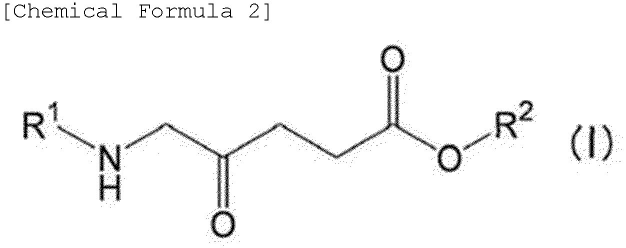 (wherein R¹ represents a hydrogen atom or an acyl group, and R² represents a hydrogen atom, a linear or branched alkyl group, a cycloalkyl group, an aryl group, or an aralkyl group) or a pharmaceutically acceptable salt or ester thereof, and
is used in combination with methotrexate or a pharmaceutically acceptable salt thereof.

Another embodiment of the present invention relates to a pharmaceutical composition for preventing and/or treating autoimmune diseases, characterized in that the pharmaceutical composition comprises methotrexate or a pharmaceutically acceptable salt thereof, and
is used in combination with a compound shown by the following Formula (I): 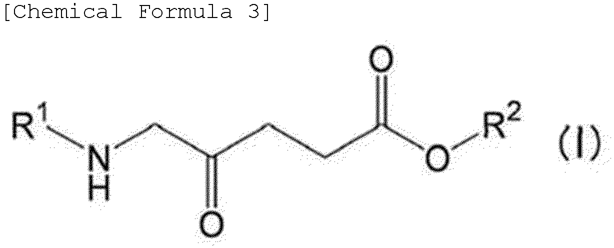 (wherein R¹ represents a hydrogen atom or an acyl group, and R² represents a hydrogen atom, a linear or branched alkyl group, a cycloalkyl group, an aryl group, or an aralkyl group)
or a pharmaceutically acceptable salt or ester thereof.

Another embodiment of the present invention relates to a medicine for preventing and/or treating autoimmune diseases comprising a combination of:
(A) a compound shown by the following Formula (I): 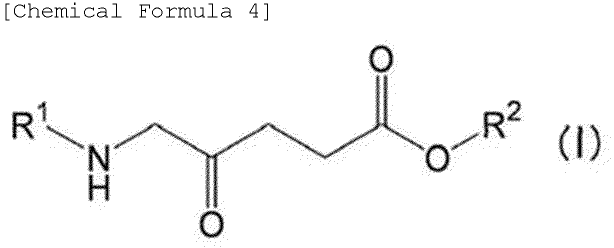 (wherein R¹ represents a hydrogen atom or an acyl group, and R² represents a hydrogen atom, a linear or branched alkyl group, a cycloalkyl group, an aryl group, or an aralkyl group) or a pharmaceutically acceptable salt or ester thereof, and
(B) methotrexate or a pharmaceutically acceptable salt thereof, that are applied to a subject at the same or different times.

One embodiment of the present invention is characterized in that in Formula (I), R¹ is selected from the group consisting of a hydrogen atom, an alkanoyl group having 1 - 8 carbons, and an aroyl group having 7 - 14 carbons, and R² is selected from the group consisting of a hydrogen atom, a linear or branched alkyl group having 1-8 carbons, a cycloalkyl group having 3 - 8 carbons, an aryl group having 6 - 14 carbons, and an aralkyl group having 7 - 15 carbons.

One embodiment of the present invention is characterized in that in Formula (I), R¹ and R² are hydrogen atoms.

One embodiment of the present invention is characterized in that the pharmaceutical composition or medicine further comprises an iron compound, or is further used in combination with an iron compound.

One embodiment of the present invention is characterized in that the iron compound is sodium ferrous citrate.

One embodiment of the present invention is characterized in that the autoimmune disease is rheumatoid arthritis or psoriasis.

Another embodiment of the present invention relates to a kit for preventing and/or treating autoimmune diseases, characterized in that is comprises:
(A) a compound shown by the following Formula (I): 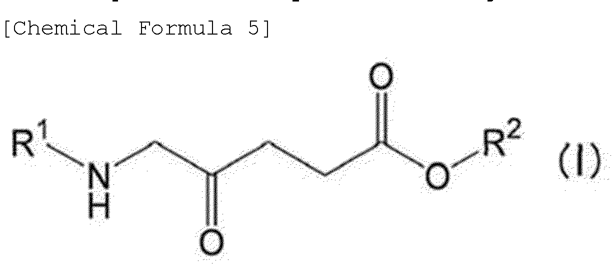 (wherein R¹ represents a hydrogen atom or an acyl group, and R² represents a hydrogen atom, a linear or branched alkyl group, a cycloalkyl group, an aryl group, or an aralkyl group) or a pharmaceutically acceptable salt or ester thereof, and
(B) methotrexate or a pharmaceutically acceptable salt thereof.

One embodiment of the present invention is characterized in that the said kit further comprises an instruction sheet comprising instructions on the dosage and administration method of the said compound shown by Formula (I) and methotrexate.

One embodiment of the present invention is characterized in that the said kit further comprises an instruction sheet comprising instructions on the dosage and administration method the said compound shown by Formula (I), methotrexate, and an iron compound.

Another embodiment of the present invention relates to the use of:
(A) a compound shown by the following Formula (I): 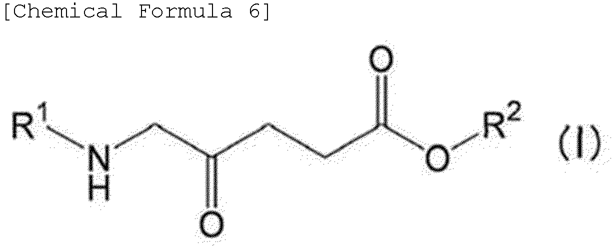 (wherein R¹ represents a hydrogen atom or an acyl group, and R² represents a hydrogen atom, a linear or branched alkyl group, a cycloalkyl group, an aryl group, or an aralkyl group)
   or a pharmaceutically acceptable salt or ester thereof, and
(B) methotrexate or a pharmaceutically acceptable salt thereof for manufacturing a medicine for preventing and/or treating autoimmune diseases.

Another embodiment of the present invention relates to a method for treating autoimmune disease, characterized in that it comprises a step of applying to a subject suffering from an autoimmune disease:
(A) a compound shown by the following Formula (I): 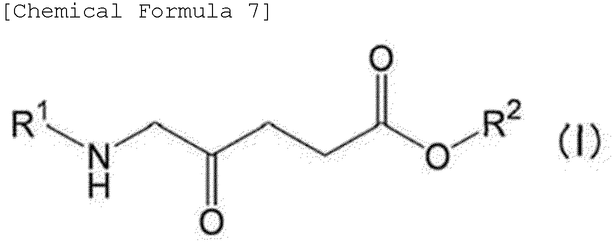 (wherein R¹ represents a hydrogen atom or an acyl group, and R² represents a hydrogen atom, a linear or branched alkyl group, a cycloalkyl group, an aryl group, or an aralkyl group) or a pharmaceutically acceptable salt or ester thereof, and
(B) methotrexate or a pharmaceutically acceptable salt thereof at the same or different times.

Note that an invention of any combination of one or more characteristics of the present invention described above is also encompassed by the scope of the present invention.

### Brief Description of the Drawings

Figure 1 shows an overview of the experiments carried out.
Figure 2 shows the change in the expression amount of each gene by application of MTX and/or 5-ALA/SFC.
Figure 3 shows the change in the expression amount of HO-1 by application of MTX and/or 5-ALA/SFC in vivo.
Figure 4 shows the results of foot volume measurement in Example 3.
Figure 5 shows the results of arthritis score measurement in Example 3.

### Description of Embodiments

The present invention relates to a combination of methotrexate (MTX) and 5-ALAs for preventing and/or treating autoimmune diseases. An autoimmune disease in the present disclosure may mean a disease that arises from the immune system attacking normal autologous cells or tissues due to an abnormality in the immune system. Exemplary autoimmune diseases can include rheumatoid arthritis or psoriasis.

In general, the IUPAC name for methotrexate is (S)-2-(4-(((2,4-diaminopteridin-6-yl)methyl)methylamino)benzamido)pentanedioic acid, and is represented by the following Formula (II).

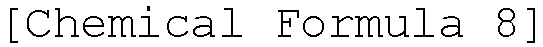

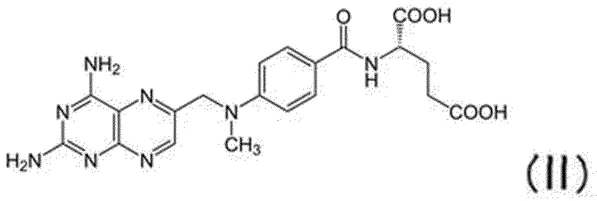

The dosage and administration route of methotrexate to a subject is not limited, and the dosage and administration route of methotrexate generally used in the treatment of autoimmune disease (such as treatment of rheumatoid arthritis or psoriasis) may be adopted. As a non-limiting example, depending on the pathology of the subject, 1 - 25 mg/week may be administered orally or by injection. Note that when the dosage is high, the above amount may be divided into 2 - 5 times a week and administered to the subject.

In the present disclosure, 5-ALAs refers to 5-ALA or derivatives thereof or salts thereof.

In the present disclosure, 5-ALA means 5-aminolevulinic acid. 5-ALA is also referred to as δ-aminolevulinic acid, and is a type of amino acid.

Compounds represented by the following Formula (I) can be exemplified as derivatives of 5-ALA. In Formula (I), R¹ represents a hydrogen atom or an acyl group, and R² represents a hydrogen atom, a linear or branched alkyl group, a cycloalkyl group, an aryl group, or an aralkyl group. Note that in Formula (I), 5-ALA corresponds to when R¹ and R² are hydrogen atoms.

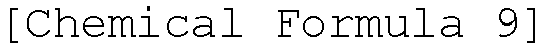

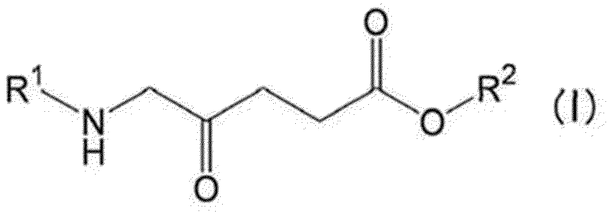

5-ALAs only need to act as an active ingredient in the state of 5-ALA or a derivative thereof of Formula (I) in the living body, and can also be administered as prodrug (precursor) that is degradated by an enzyme in the living body.

The acyl group in R¹ of Formula (I) can include a linear or branched an alkanoyl group having 1 - 8 carbons such as formyl, acetyl, propionyl, butyryl, isobutyryl, valeryl, isovaleryl, pivaloyl, hexanoyl, octanoyl, and benzylcarbonyl groups, and an aroyl group having 7 - 14 carbons such as benzoyl, 1-naphthoyl, and 2-naphthoyl groups.

The alkyl group in R² of Formula (I) can include a linear or branched alkyl group having 1-8 carbons such as methyl, ethyl, propyl, isopropyl, butyl, isobutyl, sec-butyl, tert-butyl, pentyl, isopentyl, neopentyl, hexyl, heptyl, and octyl groups.

The cycloalkyl group in R² of Formula (I) can include a cycloalkyl group having 3 - 8 carbons which is saturated or may have partially unsaturated bonds, such as cyclopropyl, cyclobutyl, cyclopentyl, cyclohexyl, cycloheptyl, cyclooctyl, cyclododecyl, and 1-cyclohexenyl groups.

The aryl group in R² of Formula (I) can include an aryl group having 6 - 14 carbons such as phenyl, naphthyl, anthryl, and phenanthryl groups.

As the aralkyl group in R² of Formula (I), the same exemplifications as the above aryl group can be made for the aryl moiety and the same exemplifications as the above alkyl group can be made for the alkyl moiety, and specifically, this can include an aralkyl group having 7 - 15 carbons such as benzyl, phenethyl, phenylpropyl, phenylbutyl, benzhydryl, trityl, naphthylmethyl, and naphthylethyl groups.

Preferred 5-ALA derivatives include compounds where R¹ is formyl, acetyl, propionyl, butyryl groups, and the like. Moreover, preferred 5-ALA derivatives include compounds where the above R² is methyl, ethyl, propyl, butyl, pentyl groups, and the like. Moreover, preferred 5-ALA derivatives include compounds where the combination of the above R¹ and R² is each combination of (formyl and methyl), (acetyl and methyl), (propionyl and methyl), (butyryl and methyl), (formyl and ethyl), (acetyl and ethyl), (propionyl and ethyl), and (butyryl and ethyl).

Among 5-ALAs, salts of 5-ALA or derivatives thereof can include pharmaceutically acceptable acid addition salts, metal salts, ammonium salts, organic amine addition salts, and the like. As acid addition salts, for example, each inorganic acid salt such as hydrochloride salts, hydrobromide salts, hydroiodide salts, phosphate salts, nitrate salts, and sulfate salts, as well as each organic acid addition salt such as formate salts, acetate salts, propionate salts, toluenesulfate salts, succinate salts, oxalate salts, lactate salts, tartrate salts, glycolate salts, methanesulfonate salts, butyrate salts, valerate salts, citrate salts, fumarate salts, maleate salts, and malate salts can be exemplified. As metal salts, each alkali metal salt such as lithium salts, sodium salts, and potassium salts, each alkaline earth metal salt such as magnesium and calcium salts, as well as each metal salt such as aluminum and zinc can be exemplified. As ammonium salts, alkyl ammonium salts such as ammonium salts and tetramethylammonium salts can be exemplified. As organic amine salts, each salt of triethylamine salts, piperidine salts, morpholine salts, and toluidine salts can be exemplified. Note that these salts may also be employed as a solution at the time of use.

Among the above 5-ALAs, the most desirable are 5-ALA, various esters such as 5-ALA methyl ester, 5-ALA ethyl ester, 5-ALA propyl ester, 5-ALA butyl ester, 5-ALA pentyl ester, 5-ALA hexyl ester, and 5-ALA heptyl ester, as well as hydrochloride salts, phosphate salts, and sulfate salt thereof. Among others, 5-ALA hydrochloride salts and 5-ALA phosphate salts can be exemplified as particularly favorable.

The above 5-ALAs can be manufactured by well-known methods such as e.g., chemical synthesis, production by microorganisms, and production by enzymes. Moreover, the above 5-ALAs may form a hydrate or a solvate, and 5-ALAs can also be employed alone or in an appropriate combination of two or more.

When the above 5-ALAs is prepared as an aqueous solution, care must be taken so that the aqueous solution does not become alkaline in order to prevent degradation of 5-ALAs. When it becomes alkaline, degradation can be prevented by removing oxygen.

Since 5-ALAs is a highly safe substance, the administration route to a subject is not limited, and various administration routes can be selected. Examples can include oral administration including sublingual administration, inhalation administration, direct administration to the target tissue or organ by a catheter, intravenous administration including injection or infusion, subcutaneous administration by a syringe, transdermal administration by e.g. a patch, suppositories, or parenteral administration such as administration by forced enteral nutrition employing nasogastric tube, nasointestinal tube, gastrostomy tube, or enterostomy tube, and the like. Oral administration can be listed as the most simple administration method.

In regard to the dosage of 5-ALAs to a subject, an optimal amount may be determined by those skilled in the art (such as a physician) depending on the objective of administration. Specifically, the dosage may be 0.1 mg - 1000 mg/day, 0.2 mg - 500 mg/day, 0.3 mg - 250 mg/day, 0.4 mg - 100 mg/day, 0.5 mg - 40 mg/day, or 0.5 mg - 20 mg/day in terms of 5-ALA per 1 kg of subject body weight.

In the present disclosure, methotrexate and 5-ALAs may be administered to a subject at the same time, or may be administered at different times. In other words, 5-ALAs may be administered at essentially the same timing as the administration of methotrexate to a subject, 5-ALAs may be administered before the administration of methotrexate to a subject, or 5-ALAs may be administered after the administration of methotrexate to a subject. Note that the administration route of methotrexate to a subject may be the same or different from the administration route of 5-ALAs to a subject. For example, when methotrexate and 5-ALAs are prepared as a drug combination, the administration routes of both agents will naturally be the same, and when methotrexate and 5-ALAs are each independently administered, the administration routes of both agents can be appropriately selected according to the pathology of the subject or the treatment policy.

5-ALAs are metabolized in the living body to become porphyrin (mainly protoporphyrin IX: PpIX). PpIX is known to have both the nature to accumulate in tissues such as tumors and the nature to be excited when a light of a given wavelength is irradiated, and can be utilized for photodynamic therapy (PDT) or photodynamic diagnosis (PDD) that utilize these natures. However, as is shown in the Examples, the present invention does not require irradiation of light ray for exerting its effect. In other words, the present invention is an invention that is based on an action mechanism that is clearly different from PDT or PDD.

In the present disclosure, 5-ALAs may be applied to a subject together with an iron compound. 5-ALAs and iron compounds are known to cooperate and exert various effects in the living body. However, since iron is a substance that is ordinarily present in the living body, if iron storage is sufficiently present in the subject body, or if the subject supplements iron by ingestion of meals or supplements, iron compound does not necessarily have to be used in combination in order to obtain the desired effects of the present invention. Non-limiting examples of iron compounds can include ferrous citrate, sodium ferrous citrate (SFC), iron sodium citrate, iron ammonium citrate, ferric pyrophosphate, heme iron, iron dextran, iron lactate, ferrous gluconate, DTPA iron, iron sodium diethylenetriaminepentaacetate, iron ammonium diethylenetriaminepentaacetate, iron sodium ethylenediaminetetraacetate, iron ammonium ethylenediaminepentaacetate, triethylenetetramine iron, iron sodium dicarboxymethylglutamate, iron ammonium ammonium dicarboxymethylglutamate, lactoferrin iron, transferrin iron, ferric chloride, iron sesquioxide, sodium iron chlorophyllin, ferritin iron, ferrous fumarate, ferrous pyrophosphate, saccharated iron oxide, iron acetate, iron oxalate, ferrous succinate, iron sodium succinate citrate, iron sulfate, and iron glycine sulfate.

The dosage of the iron compound to a subject may be 0 - 100-folds in molar ratio to the dosage of 5-ALAs to the subject, desirably 0.01-folds - 10-folds, and more desirably 0.05-folds - 5-folds.

The terms used herein, except for those that are particularly defined, are employed for describing particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

The present invention will now be described in further detail with reference to Examples. However, the present invention can be embodied by various aspects, and shall not be construed as being limited to the Examples described herein.

### Examples

### Example 1: In Vitro Experiments

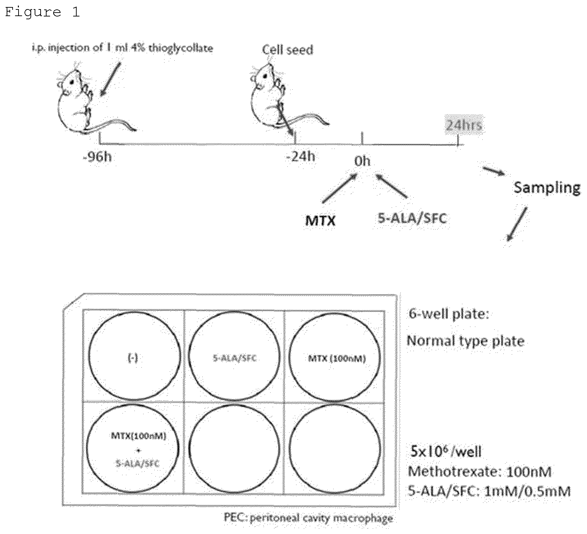

To 8 weeks-old female C57BL/6NCrSlc(B6/N) mice, 1 ml of 4% thioglycolate (70157-100G, Sigma-Aldrich) was intraperitoneally administered. After 72 hours, peritoneum macrophage was collected, and this was cultured using MEM α medium supplemented with 10% FBS under a condition of 5% CO₂ and 95% air at 37°C for 24 hours. Stimulation with different concentrations of MTX (M8407-100MG/Sigma-Aldrich Co. LLC) (100 nM) and/or 5-ALA hydrochloride salt/SFC (KOMATSUYA CORPORATION) (1 mM/0.5 mM) was performed for 24 hours. These were plated in 2 ml 6-well tissue culture plates (Greiner Bio-One) at 5 × 10⁶ cells/well (Figure 1).

Next, RNA samples were extracted from the cultured cells, and oligo (dT) primers and Super Script reverse transcription enzyme (Invitrogen) were employed for reverse transcription into cDNA. Gene expression was analyzed by qRT-PCR on Applied Biosystem PRISM 7700 device (Applied Biosystems) with TaqMan system (18S and Arg1) and SYBR Green system (18s, HO-1, CD206, and IL-10). The threshold cycle (Ct) values of target genes were normalized with the Ct value of 18S rRNA. The final result was analyzed with the ΔΔCt method. The sequences of the target-specific primers and probes are shown in Table 1 and Table 2.

**[Table 1]**

| Table 1: Primer and Probe Sequences employed in the TaqMan System | | | |
|---|---|---|---|
| Genes | Forward (5'-3') primers | Reverse (5'-3') primers | Probe |
| 18s | ATGAGTCCACTTTAAA TCCTTTAACGA (SEQ ID NO. 1) | CTTTAATATACGCTA TTGGAGCTGGAA (SEQ ID NO. 2) | ATCCATTGGAGGGCA AGTCTGGTGC (SEQ ID NO. 3) |
| Arg1 | TGCTGCAGGGCCTTT CTC (SEQ ID NO. 4) | GAGCTCCAAGCCAA AGTCCTT (SEQ ID NO. 5) | CCTCCTCGAGGCTGT CCTTTTGAGAAAG (SEQ ID NO. 6) |

**[Table 2]**

| Table 2: Primer Sequences Employed in the SYBR Green System | | |
|---|---|---|
| Genes | Forward (5'-3') primers | Reverse (5'-3') primers |
| 18s | ATGAGTCCACTTTAAATCCTTTAA CGA (SEQ ID NO. 7) | CTTTAATATACGCTATTGGAGCTGG AA (SEQ ID NO. 8) |
| HO-1 | GAAATCATCCCTTGCACGCC (SEQ ID NO. 9) | CCTGAGAGGTCACCCAGGTA (SEQ ID NO. 10) |
| CD106 | CAAGGAAGGTTGGCATTTGT (SEQ ID NO. 11) | CCTTTCAGTCCTTTGCAAGC (SEQ ID NO. 12) |
| IL-10 | GCTCTTACTGACTGGCATGAG (SEQ ID NO. 13) | CGCAGCTCTAGGAGCATGTG (SEQ ID NO. 14) |

### Statistical Analysis Method of Experimental Results

The results are represented in average value and standard deviation (average value ± SD). For statistical analysis, GraphPad Prism (version 7.0, GraphPad Software, San Diego, CA) was employed for analysis. One-sided Student's t-test was used for comparison of two unpaired groups. For comparison of multiple groups, one-way analysis of variance (One-way ANOVA) with Tukey's multiple comparison test was employed.
*, **, ***, **** in the Figures each mean the following.
*: p < 0.05
**: p < 0.01
***: p < 0.001
****: p < 0.0001

### Results

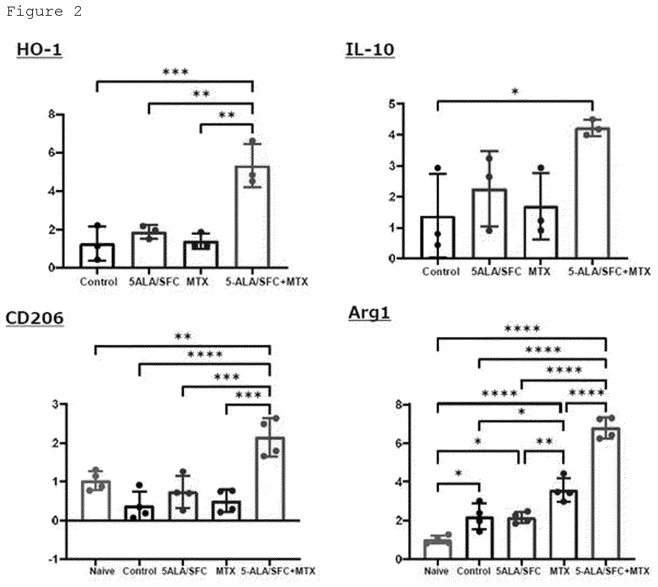

The results of the qRT-PCR gene analysis are shown in Figure 2. By applying both MTX and 5-ALA/SFC, the expression amount of heme oxygenase 1 (HO-1) was significantly increased compared to the control group/5-ALA/SFC alone administration group/MTX alone administration group.

HO-1 is an enzyme that catalyzes the first rate-limiting step of heme degradation, and in most tissues, the expression of this enzyme in an ordinary state is low or does not exist. However, it is known that in inflamed tissues, HO-1 is expressed at a high level and a strong anti-inflammatory effect is exhibited. Accordingly, from the results of this experiment, it is suggested that synergistic anti-inflammatory effect is exerted by combining MTX and 5-ALA/SFC.

Further, as shown in Figure 2, by applying both MTX and 5-ALA/SFC, the expression of M2 macrophage-related factors IL-10, CD206, and Arg1 were also significantly increased. M2 macrophage is known to be a factor that acts mainly on controlling inflammation. Accordingly, from these results, it was further supported that synergistic anti-inflammatory effect is exerted by combining MTX and 5-ALA/SFC.

### Example 2: In Vivo Experiments

To 8 weeks-old female C57BL/6NCrSlc(B6/N) mice, 1 ml of 4% thioglycolate (70157-100G, Sigma-Aldrich) was intraperitoneally administered. The mice were then divided into the control group, the 5-ALA/SFC group, the MTX group, and the 5-ALA/SFC + MTX group. Nothing was administered to the control group. For the 5-ALA/SFC group, 4% thioglycolate was administered, immediately after, 24 hours after, or 48 hours after which oral administration of 100 mg/kg of 5-ALA hydrochloride salt and 157 mg/kg of SFC (KOMATSUYA CORPORATION) was performed. For the MTX group, 4% thioglycolate was administered, immediately after which 10 mg/kg of MTX (M8407-100MG/Sigma-Aldrich Co. LLC) was subcutaneously injected. For the 5-ALA/SFC + MTX group, both the said oral administration of 5-ALA/SFC and the said subcutaneous injection of MTX were carried out. Note that the group without administration of any of thioglycolate, 5-ALA hydrochloride salt, SFC, and MTX was set as the Naive group.

After collecting cells in the peritoneal fluid 72 hours after administering 4% thioglycolate, a flow cytometer (FACSymphony^{™} A3; Becton Dickinson) was employed for staining with Live/Dead stain (L34957, Thermo Fisher Scientific), anti-CD11b-FITC antibody (BioLegend), and anti-HO-1-PE antibody (ab83214; Abcam), and this was analyzed with FlowJo software (Version 10.5.0; BD Biosciences). After gating out the dead cells, HO-1 expression in each group was compared. The statistical analysis method of experimental results was the same as the statistical analysis method employed in Example 1.

### Results

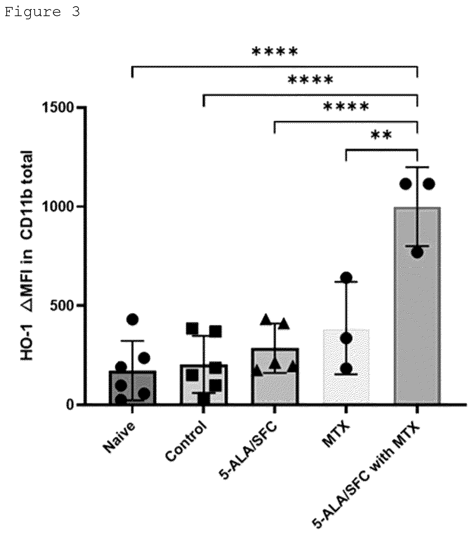

The results of mean fluorescent intensity (MFI) by a flow cytometer are shown in Figure 3. From this result, it can be seen that expression of HO-1 increased significantly by the use of MTX with 5-ALA and SFC compared to the subject group having only 4% thioglycolate administrated. Surprisingly, the use of MTX with 5-ALA and SFC in combination resulted in an increase in expression amount of HO-1 that exceeds the additive extent. From this, it was shown that the use of MTX with 5-ALA and SFC in combination synergistically increases the expression amount of HO-1. Accordingly, from these results, it was further supported that synergistic anti-inflammatory effect is exerted by combining MTX and 5-ALA/SFC.

### Discussion

MTX is an immunosuppressing agent having folic acid metabolism antagonizing mechanism, and is an agent that is broadly used worldwide as a therapeutic drug for autoimmune diseases (in particular, rheumatoid arthritis and psoriasis). Here, as shown in the results of this Example, it was shown that synergistic anti-inflammatory effect is exerted by the combination of MTX and 5-ALA/SFC. In other words, synergistically elevated therapeutic effect on autoimmune diseases is expected by the combination of MTX and 5-ALA/SFC.

### Example 3: Experiments Employing Collagen-Induced Arthritis (CIA) Model

For 9 weeks-old female DA/Slc rats, under isoflurane anesthesia employing a simplified inhalation anesthesia device for animals (model type: NARCOBIT-E (type II), Natsume Seisakusho Co.,Ltd.), the tail root which is the administration site was shaved with an electric shaver, and 0.05 mL in each of left and right sides of the tail root of all animals for a total of 0.1 mL of the symptom evoking substance administration fluid was intracutaneously administrated with a syringe (Terumo Corporation) and an injection needle (Terumo Corporation). The symptom evoking substance administration fluid was prepared by adding bovine type-II collagen and an equal amount of Incomplete Freund Adjuvant to an interchangeable glass syringe (TOP Corporation), and then mixing well.

The symptom evoking substance was administered only on Day 0, and each test substance was administered before the administration of the symptom evoking substance on the first day. MTX was suspended in 0.5 w/v% methylcellulose 400 solution (MC). ALA employed was aminolevulinic acid hydrochloride, and weights shown below are weights as aminolevulinic acid hydrochloride.

The rats were divided into Groups 1 - 5 (n = 10 for each group), and each test substance was orally administered with an oral sonde (Fuchigami Kikai Co., Ltd.) and a syringe (Terumo Corporation) once a day for 18 consecutive days. The daily dosage is as shown below.
[Group 1] MTX : 0.02 mg/kg
[Group 2] MTX : 0.02 mg/kg + ALA: 100 mg/kg + SFC: 157 mg/kg
[Group 3] ALA: 100 mg/kg + SFC: 157 mg/kg
[Group 4] ALA: 100 mg/kg
[Group 5 (Control group)] MC: 10 ml/kg

For evaluating symptoms, increase in foot volume and scoring of extent of macroscopic arthritis were employed.

For measurement of foot volume, the foot volumes of left and right posterior limbs were each measured on Days 0 (before grouping), 4, 7, 11, 14, and 18 with a Plethysmometer (model type: MK-101P, Muromachi Kikai Co., Ltd.). The foot volume was measured three times per foot, and the average value of the three was calculated. The foot volume of the other foot was similarly measured. The foot volume measurement value of one individual was calculated with the following mathematical formula. [Foot volume measurement value = (Average value of right foot + Average value of left foot) / 2]

The measurement value before grouping was set as the Pre-value and the measurement value at each measurement point after grouping was set as the Post-value, and the value of increase in foot volume (Δ mL) was calculated by Post-value - Pre-value for each individual.

The arthritis score measurement was measured on each of Days 0 (before grouping), 4, 7, 11, 14, and 18. The condition of the left and right posterior and anterior limbs was each observed, and evaluated as 0 point score for no symptom, 1 point score when swelling of one small joint such as a digit is seen, 2 points score when swelling of two or more small joints or a large joint is seen, 3 points score when swelling of one limb is seen, and 4 points score when maximum swelling is seen in the entirely of one limb. Note that the total sum of left and right arthritis scores was calculated as the score for that individual.

Moreover, for foot volumes and scores, group average value ± standard error (Mean ± S.E) was calculated to perform statistical processing by a spreadsheet software Excel (Microsoft Corporation). Intergroup comparison was performed by Student's t-test with one-sided significance level of 5%. Moreover, the following were employed for determination of synergistic effect: whether or not the intergroup difference of average values between Group 1 and Group 5 exceeds the sum value of the intergroup difference between Group 1 and Group 5 and the intergroup difference between Group 3 and Group 5, or, using Colby's Equation (theoretical value E = A + B - (A × B) / 100), whether the percentage of intergroup difference of average values between Group 2 and Group 5 exceeds the theoretical value (E) determined from the percentage of intergroup difference between Group 1 and Group 5 (A) and the percentage of intergroup difference between Group 3 and Group 5 (B) .

### Results

### (1) Value of increase in foot volume

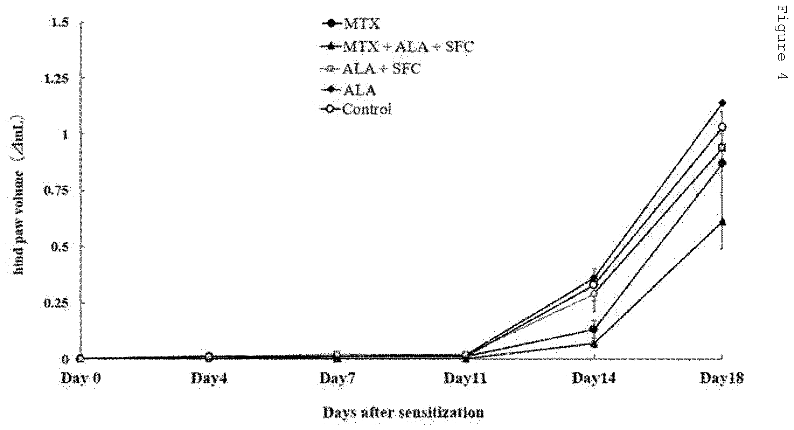

The results of foot volume measurement are shown in Figure 4. On Day 18, the last measurement day, the intergroup difference between Group 2 and Group 5 (0.42 mL) was larger than the sum value (0.25 mL) of the intergroup difference between Group 1 and Group 5 (0.16 mL) and the intergroup difference between Group 3 and Group 5 (0.09 mL). Moreover, percentage of decrease of Group 2 against Group 5 (40.8%) was larger than the theoretical value determined from Colby's Equation (24.2%). Further, as a result of comparing Group 2 with Group 5 and Group 2 with Group 3 on Day 18, Group 2 was significantly lower in all cases. Moreover, the intergroup difference between Group 1 and Group 2 had a trend of Group 2 being lower with p = 0.08. In other words, a surprising synergistic symptom suppression effect that exceeds the additive effect predicted from the effects of each agent alone was seen by the combination of MTX and 5-ALA/SFC.

### (2) Arthritis score

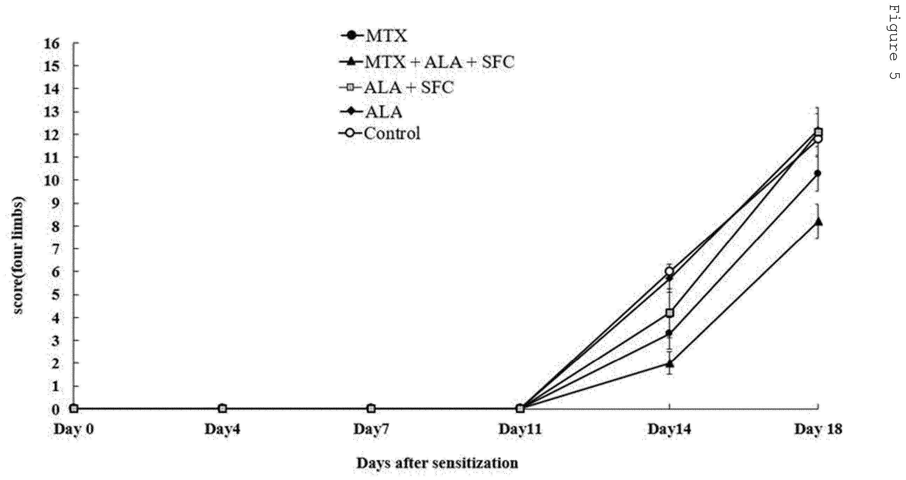

The results of arthritis score measurement are shown in Figure 5. On Day 18, the last measurement day, the intergroup difference between Group 2 and Group 5 (3.6) was larger than the sum value (1.5) of the intergroup difference between Group 1 and Group 5 (1.5) and the intergroup difference between Group 3 and Group 5 (0.0) (worse than the control). Moreover, percentage of decrease of Group 2 against Group 5 (30.5%) was larger than the theoretical value determined from Colby's Equation (12.7%). Further, as a result of comparing Group 2 with Group 5, Group 1 with Group 2, and Group 2 with Group 3 on Day 18, Group 2 was significantly lower in all cases. In other words, a surprising synergistic arthritis score decreasing effect that exceeds the additive effect predicted from the effects of each agent alone was seen by the combination of MTX and 5-ALA/SFC.

### Discussion

In this Example, it was confirmed that synergistic anti-inflammatory effect is exerted by the combination of MTX and 5-ALA/SFC in experiments employing collagen-induced arthritis (CIA) model rats as well. Note that as previously described, MTX is an immunosuppressing agent having folic acid metabolism antagonizing mechanism, and is an agent that is broadly used worldwide as a therapeutic drug for autoimmune disease (in particular, rheumatoid arthritis and psoriasis). In this Example as well, a certain level of anti-inflammatory effect was seen in the group having only MTX administrated (Group 1), and this result shows that this experimental system is an appropriate experimental system.

## Claims

1. A pharmaceutical composition for preventing and/or treating autoimmune diseases, **characterized in that** it comprises:
(A) a compound shown by the following Formula (I): 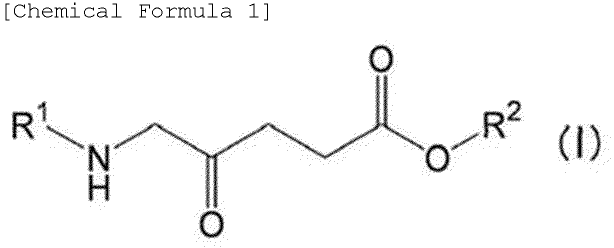 (wherein R¹ represents a hydrogen atom or an acyl group, and R² represents a hydrogen atom, a linear or branched alkyl group, a cycloalkyl group, an aryl group, or an aralkyl group) or a pharmaceutically acceptable salt or ester thereof, and
(B) methotrexate or a pharmaceutically acceptable salt thereof.

2. A pharmaceutical composition for preventing and/or treating autoimmune diseases, **characterized in that** it comprises:
a compound shown by the following Formula (I): 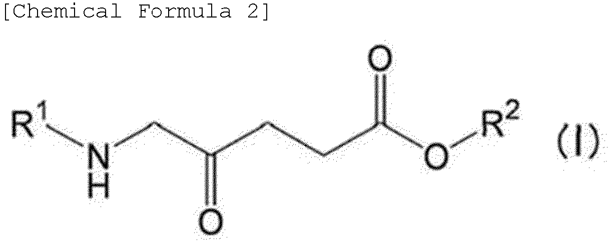 (wherein R¹ represents a hydrogen atom or an acyl group, and R² represents a hydrogen atom, a linear or branched alkyl group, a cycloalkyl group, an aryl group, or an aralkyl group) or a pharmaceutically acceptable salt or ester thereof, and
is used in combination with methotrexate or a pharmaceutically acceptable salt thereof.

3. A pharmaceutical composition for preventing and/or treating autoimmune diseases, **characterized in that** it comprises methotrexate or a pharmaceutically acceptable salt thereof, and
is used in combination with a compound shown by the following Formula (I): 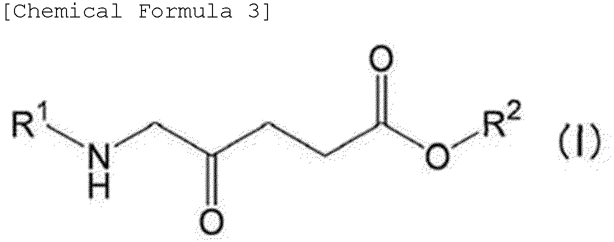 (wherein R¹ represents a hydrogen atom or an acyl group, and R² represents a hydrogen atom, a linear or branched alkyl group, a cycloalkyl group, an aryl group, or an aralkyl group) or a pharmaceutically acceptable salt or ester thereof.

4. A medicine for preventing and/or treating autoimmune diseases comprising a combination of:
(A) a compound shown by the following Formula (I): 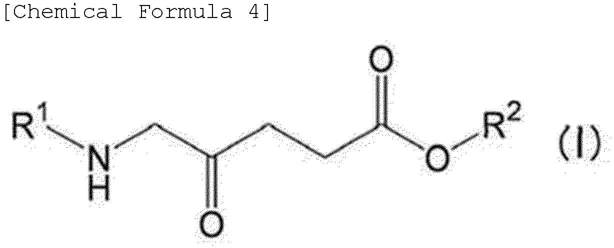 (wherein R¹ represents a hydrogen atom or an acyl group, and R² represents a hydrogen atom, a linear or branched alkyl group, a cycloalkyl group, an aryl group, or an aralkyl group) or a pharmaceutically acceptable salt or ester thereof, and
(B) methotrexate or a pharmaceutically acceptable salt thereof, that are applied to a subject at the same or different times.

5. The pharmaceutical composition or medicine according to any one of claims 1 to 4, **characterized in that**:
R¹ is selected from the group consisting of a hydrogen atom, an alkanoyl group having 1 - 8 carbons, and an aroyl group having 7 - 14 carbons, and
R² is selected from the group consisting of a hydrogen atom, a linear or branched alkyl group having 1-8 carbons, a cycloalkyl group having 3 - 8 carbons, an aryl group having 6 - 14 carbons, and an aralkyl group having 7 - 15 carbons.

6. The pharmaceutical composition or medicine according to claim 5, **characterized in that** R¹ and R² are hydrogen atoms.

7. The pharmaceutical composition or medicine according to any one of claims 1 to 4, **characterized in that** it further comprises an iron compound, or is further used in combination with an iron compound.

8. The pharmaceutical composition or medicine according to claim 7, **characterized in that** the said iron compound is sodium ferrous citrate.

9. The pharmaceutical composition or medicine according to any one of claims 1 to 4, **characterized in that** the autoimmune disease is rheumatoid arthritis.

10. The pharmaceutical composition or medicine according to any one of claims 1 to 4, **characterized in that** the autoimmune disease is psoriasis.

11. A kit for preventing and/or treating autoimmune diseases, **characterized in that** is comprises:
(A) a compound shown by the following Formula (I): 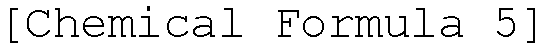 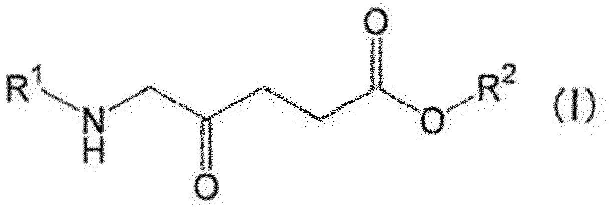 (wherein R¹ represents a hydrogen atom or an acyl group, and R² represents a hydrogen atom, a linear or branched alkyl group, a cycloalkyl group, an aryl group, or an aralkyl group) or a pharmaceutically acceptable salt or ester thereof, and
(B) methotrexate or a pharmaceutically acceptable salt thereof.

12. The use of:
(A) a compound shown by the following Formula (I): 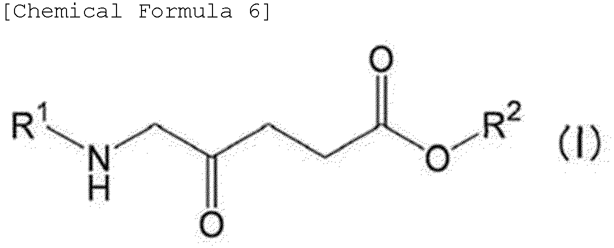 (wherein R¹ represents a hydrogen atom or an acyl group, and R² represents a hydrogen atom, a linear or branched alkyl group, a cycloalkyl group, an aryl group, or an aralkyl group) or a pharmaceutically acceptable salt or ester thereof, and
(B) methotrexate or a pharmaceutically acceptable salt thereof for manufacturing a medicine for preventing and/or treating autoimmune diseases.

13. A method for treating autoimmune disease, **characterized in that** it comprises a step of applying to a subject suffering from an autoimmune disease:
(A) a compound shown by the following Formula (I): 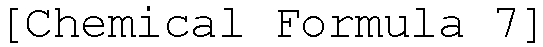 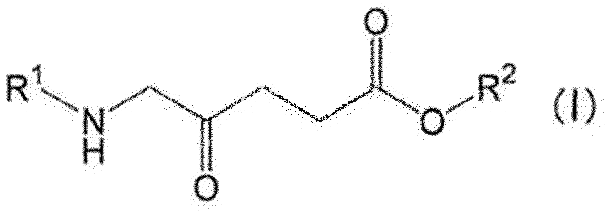 (wherein R¹ represents a hydrogen atom or an acyl group, and R² represents a hydrogen atom, a linear or branched alkyl group, a cycloalkyl group, an aryl group, or an aralkyl group) or a pharmaceutically acceptable salt or ester thereof, and
(B) methotrexate or a pharmaceutically acceptable salt thereof at the same or different times.
